# EUROPEAN PATENT APPLICATION

(11) **EP 4 249 580 A1**
(43) Date of publication of application: **27.09.2023**
(21) Application number: 21894482.5
(22) Date of filing: 04.11.2021
(51) Int. Cl.: C12M 1/00, C12Q 1/68

(54) **TREATMENT APPARATUS, NUCLEIC ACID EXTRACTION SYSTEM, AND NUCLEIC ACID ANALYSIS SYSTEM**

(30) Priority: 19.11.2020 JP 2020192547
(71) Applicant: Yokogawa Electric Corporation, Musashino-shi Tokyo 180-8750 (JP)
(72) Inventor: KUWATA Masahiro, Musashino-shi, Tokyo 180-8750 (JP)
(74) Representative: Winter, Brandl - Partnerschaft mbB
(86) International application number: PCT/JP2021/040655
(87) International publication number: WO 2022/107608

(57) **Abstract**

A treatment apparatus includes a heating block (11) configured to heat a container (30) accommodating a sample (100) including a liquid within a first range (11A) in a gravity direction, and a cooling block (12) configured to cool the container (30) heated by the heating block (11) within a second range (12A) narrower than the first range (11A) in the gravity direction.

## Description

### [Technical Field]

The present invention relates to a treatment apparatus, a nucleic acid extraction system, and a nucleic acid analysis system.

### [Background Art]

In order to extract nucleic acid from cells, there is a need to destroy (dissolve) membrane structures of cells and release contents of the cells to the outside of the cells. The following Patent Document 1 discloses a method for extracting nucleic acid from cells by performing treatment of a container accommodating a sample including a liquid at a high temperature of 100°C or higher. The following Patent Document 2 discloses an automatic execution device for a polymerase chain reaction performing heating and cooling with respect to a container accommodating a sample including a liquid with a single sample block.

### [Citation List]

### [Patent Document]

[Patent Document 1]
   Japanese Patent No. 5624487
[Patent Document 2]
   Japanese Unexamined Patent Application Publication No. 2011-19537

### [Summary of Invention]

### [Technical Problem]

In order for a user to be able to handle a container after heating treatment has ended, there is a need to lower the temperature thereof to nearly an ordinary temperature. However, Patent Documents 1 and 2 do not mention how to deal with condensation of vapor inside a container at that time. It is difficult to collect droplets which have adhered to a container wall surface due to condensation of vapor with a pipette or the like, and this may cause decrease in the amount of sample. Moreover, if droplets adhere to a lid or the like of a container, there is a probability that the droplets will scatter when the lid is opened, and there is concern that the droplets may be incorporated into samples in other containers and cause contamination.

The present invention has been made in consideration of the foregoing circumstances, and an object thereof is to provide a treatment apparatus, a nucleic acid extraction system, and a nucleic acid analysis system, in which adhesion of droplets to a container wall surface due to condensation of vapor can be curbed.

### [Solution to Problem]

(1) A treatment apparatus according to an aspect of the present invention may include: a heating block configured to heat a container accommodating a sample including a liquid within a first range in a gravity direction; and a cooling block configured to cool the container heated by the heating block within a second range narrower than the first range in the gravity direction.
(2) In the treatment apparatus according to the foregoing (1), the second range may include a lower portion of the container.
(3) In the treatment apparatus according to the foregoing (1) or (2), the second range may include a part on a side above a liquid level of the sample in the container.
(4) In the treatment apparatus according to the foregoing (1) or (2), the second range may be on only a side below a liquid level of the sample in the container.
(5) In the treatment apparatus according to any one of the foregoing (1) to (4), a first insertion hole into which the container is inserted and heated within the first range may be formed in the heating block. A second insertion hole into which the container is inserted and cooled within the second range may be formed in the cooling block. The second insertion hole may be shallower than the first insertion hole.
(6) The treatment apparatus according to any one of the foregoing (1) to (5) may further include a second cooling block configured to cool the container cooled by the cooling block within a third range wider than the second range in the gravity direction.
(7) The treatment apparatus according to the foregoing (5) may further include a second cooling block configured to cool the container cooled by the cooling block within a third range wider than the second range in the gravity direction. A third insertion hole into which the container is inserted and cooled within the third range may be formed in the second cooling block. The third insertion hole may be deeper than the second insertion hole.
(8) In the treatment apparatus according to the foregoing (5), the container may include a container main body which has a plurality of accommodation portions accommodating the sample and coupling portions coupling the plurality of accommodation portions to each other.
(9) In the treatment apparatus according to the foregoing (8), an inner wall surface of the first insertion hole may be in contact with or close to an insertion part excluding upper end portions of the plurality of accommodation portions.
(10) In the treatment apparatus according to the foregoing (8) or (9), the container may include a lid which has a lid portion covering upper end opening portions of the plurality of accommodation portions and a plurality of sealing portions inserted into the upper end opening portions.
(11) In the treatment apparatus according to the foregoing (10), the lid portion may have a flat plate shape having the same size as the coupling portions.
(12) In the treatment apparatus according to the foregoing (10) or (11), the plurality of sealing portions may be a plurality of projecting portions protruding downward from a lower surface of the lid portion.
(13) In the treatment apparatus according to the foregoing (12), an annular groove having a sealing material disposed therein may be formed on a circumferential surface of a lower end portion of each of the plurality of sealing portions.
(14) In the treatment apparatus according to the foregoing (13), the sealing material may seal by abutting an inner wall surface of each of the plurality of accommodation portions.
(15) In the treatment apparatus according to the foregoing (14), the sealing material may be a material having a lower modulus of elasticity than at least one of the plurality of accommodation portions or the lid portion.
(16) The treatment apparatus according to the foregoing (10) may further include a movement device configured to sequentially move the container to the heating block and then the cooling block. The movement device may include a container mounting portion in which the container is mounted. The container mounting portion may include a container support portion configured to support the coupling portions in a state in which the plurality of accommodation portions are suspended, and a container fixing portion configured to overlap the lid portion. The container fixing portion may be configured to sandwich the container together with the container support portion. The container fixing portion may be provided in a manner of being able to be opened and closed with respect to the container support portion.
(17) In the treatment apparatus according to the foregoing (16), the container fixing portion may have a flat plate shape having the same size as the container support portion.
(18) In the treatment apparatus according to the foregoing (16) or (17), the container fixing portion may include a hook portion capable of being engaged and disengaged with respect to the container support portion. The container fixing portion may switch between a locked state in which the container fixing portion is unable to be released with respect to the container support portion, and an unlocked state in which the container fixing portion is able to be released with respect to the container support portion.
(19) A nucleic acid extraction system according to another aspect of the present invention may include the treatment apparatus according to any one of the foregoing (1) to (18). The nucleic acid extraction system may extract nucleic acid from cells of the sample.
(20) A nucleic acid analysis system according to another aspect of the present invention may include the nucleic acid extraction system according to the foregoing (19). The nucleic acid analysis system may analyze the extracted nucleic acid.

### [Advantageous Effects of Invention]

According to an aspect of the present invention described above, it is possible to obtain a treatment apparatus, a nucleic acid extraction system, and a nucleic acid analysis system, in which adhesion of droplets to a container wall surface due to condensation of vapor can be curbed.

### [Brief Description of Drawings]

FIG. 1 is a schematic view of a nucleic acid analysis system according to a first embodiment.
FIG. 2 is a schematic view of a nucleic acid extraction system according to the first embodiment.
FIG. 3 is a cross-sectional view of a heating block according to the first embodiment.
FIG. 4 is a cross-sectional view of a cooling block according to the first embodiment.
FIG. 5 is a schematic view of the nucleic acid extraction system according to a second embodiment.
FIG. 6 is a cross-sectional view of a second cooling block according to the second embodiment.
FIG. 7 is a cross-sectional view of the cooling block according to a third embodiment.
FIG. 8 is a cross-sectional view of the cooling block according to a fourth embodiment.

### [Description of Embodiments]

Hereinafter, a treatment apparatus, a nucleic acid extraction system, and a nucleic acid analysis system according to embodiments of the present invention will be described in detail with reference to the drawings. In the following description, an overview of the embodiments of the present invention will be described first, and details of the embodiments of the present invention will be described subsequently.

### [Overview]

In the foregoing automatic execution device for a polymerase chain reaction disclosed in Patent Document 2, double strands of nucleic acid (DNA) in a sample are separated into single strands by inserting a container accommodating a sample including a liquid into a sample block and heating the sample block using an electrothermal heater or the like. Thereafter, a refrigerant is caused to flow in the same sample block to cool the sample, and the nucleic acid (DNA) is thereby replicated due to specific binding and elongation caused by the subsequent reaction. However, since a container wall surface is also cooled at the same time, vapor generated at the time of heating is condensed and adheres to the inside of the container as droplets.

In addition, in the foregoing nucleic acid extraction method disclosed in Patent Document 1, bacteria or fungi are caused to be in a high-temperature state exceeding the boiling point inside a container and a high-pressure state realized by a saturated vapor pressure in the high-temperature state, and the nucleic acid is extracted from the bacteria or fungi. Therefore, condensation of vapor at the time of cooling the container in this case becomes more prominent than that in the case of the foregoing Patent Document 2 in which heat treatment is performed at a temperature near the boiling point.

Unless droplets which have adhered to the container wall surface are collected in a lower portion of a container, for example, by means of a centrifugal force or the like of a centrifuge, it is difficult to move them to other containers using a pipette or the like, and this may cause decrease in the amount of sample. Moreover, if droplets adhere to a lid or the like of a container, there is a probability that the droplets will scatter when the lid is opened, and there is concern that the droplets may be incorporated into samples in other containers and cause contamination. Particularly in recent years, with the increasing scale and advancement of analysis using nucleic acid, there has come to be cases in which a plurality of containers are arrayed in a column direction, a row direction, or both directions and treatment is performed with respect to a plurality of samples at the same time. Therefore, contamination due to scattering of droplets poses a serious problem.

According to the embodiments of the present invention, in a treatment apparatus, a nucleic acid extraction system, and a nucleic acid analysis system, a container accommodating a sample including a liquid is heated within a first range in a gravity direction by a heating block, and the container heated by the heating block is cooled within a second range narrower than the first range in the gravity direction by a cooling block. Accordingly, the range of cooling a container becomes narrower than the range of heating the container, and therefore adhesion of droplets to a container wall surface due to condensation of vapor can be curbed.

### [First embodiment]

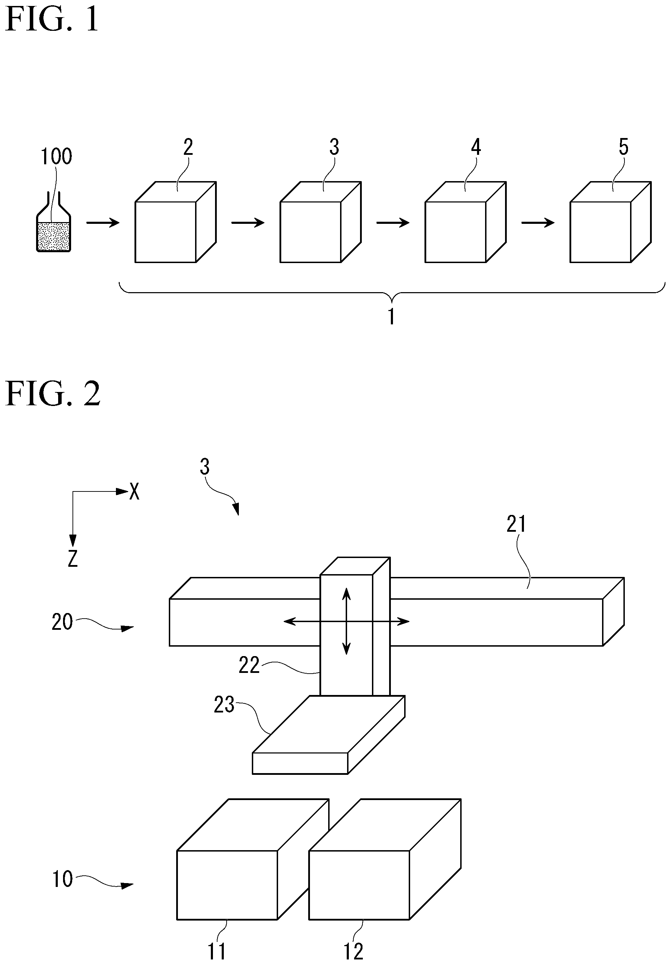

FIG. 1 is a schematic view of a nucleic acid analysis system 1 according to a first embodiment.

As illustrated in FIG. 1, the nucleic acid analysis system 1 includes a bacteria collection system 2, a nucleic acid extraction system 3, a hybridization reaction system 4, and a detection system 5.

The bacteria collection system 2 is a system for collecting bacteria (bacteria, fungi, or the like) included in a sample 100 from the sample 100. For example, in the case of an examination targeted on a beverage, the sample 100 is a manufactured beverage, water for manufacturing the beverage, a liquid in a process of manufacturing the beverage, or the like. Alternatively, the sample 100 may be a liquid obtained while collecting bacteria from a cotton swab or the like which has wiped up in an examination environment in order to examine for the presence or absence of bacterial contamination or the degree of contamination in a manufacturing environment.

For example, bacteria can be collected through filtering using a filter by applying compression or decompression to a collected liquid. For example, regarding a filter, in a case of collecting bacteria or fungi, it is preferable to use a filter having a pore diameter of 0.22 µm to 0.45 µm. After bacteria are collected using a filter, the filter may be immersed into a culture solution in which the bacteria are cultured, and the culture solution in which the bacteria are cultured may be used as the sample 100 in the next step (nucleic acid extraction system 3). Alternatively, a liquid in which bacteria are collected through centrifugal separation or the like, or a liquid in which an aggregate collected through centrifugal separation or the like has been dissolved may be used as the sample 100 in the next step. Alternatively, a liquid having a filter therein may be vibrated, and a liquid including suspended bacteria may be used as the sample 100 in the next step.

The nucleic acid extraction system 3 is a system for destroying (dissolving) membrane structures of cells in the sample 100 and extracting the nucleic acid of the cells. A liquid including a different nucleic acid reacting with the extracted nucleic acid may be mixed into the sample 100 with the extracted nucleic acid. In addition, the different nucleic acid may be a nucleic acid to which a part having fluorescence, luminescence, or a quenching action has been imparted under particular conditions to be used for detection in a detecting step (detection system 5), which will be described below. These may be mixed into the sample 100 before treatment is performed in the nucleic acid extraction system 3 or may be mixed into the sample 100 after treatment is performed in the nucleic acid extraction system 3.

The hybridization reaction system 4 is a system for causing a hybridization reaction in the nucleic acid in the sample 100. In this step, for example, the sample 100 is heated to 60°C and agitated so that the foregoing hybridization reaction consistent with a different nucleic acid occurs. In this reaction, for example, the foregoing part having fluorescence, luminescence, or a quenching action under particular conditions and having been imparted to a different nucleic acid reacts with the nucleic acid in the sample 100, and therefore fluorescence, luminescence, or a quenching action is manifested.

By designing the foregoing structure of a different nucleic acid to react with a particular nucleic acid, for example, it can react with only the nucleic acid possessed by particular bacteria, fungi, or the like in the sample 100. Namely, in treatment of the hybridization reaction system 4, by using a different nucleic acid reacting with a particular nucleic acid, fluorescence, luminescence, or a quenching action imparted to the different nucleic acid can be manifested only when particular bacteria, fungi, or the like are included in the sample 100.

The detection system 5 detects the presence or absence, the degree, and the like of fluorescence, luminescence, or a quenching action manifested in the sample 100 subjected to treatment by the hybridization reaction system 4. For example, the detection system 5 excites the fluorescence action manifested in the nucleic acid in the sample 100 using an excitation laser beam and detects excited fluorescence using a high-sensitivity camera.

Alternatively, the detection system 5 detects the luminescence action manifested in the nucleic acid in the sample 100 using a high-sensitivity camera. Alternatively, regarding the quenching action manifested in the nucleic acid in the sample 100, the detection system 5 detects the degree of extinction of fluorescence or luminescence imparted to a part in the vicinity of the part to which the quenching action is imparted, using a high-sensitivity camera. For example, regarding this detection method, such a method disclosed in Japanese Unexamined Patent Application, First Publication No. 2020-74726 may be employed.

The nucleic acid analysis system 1 analyzes whether particular bacteria (bacteria, fungi, or the like) are included in the sample 100 or analyzes the concentration thereof using a series of systems described above.

FIG. 2 is a schematic view of the nucleic acid extraction system 3 according to the first embodiment.

As illustrated in FIG. 2, the nucleic acid extraction system 3 is provided with a treatment apparatus including a heat treatment device 10 and a movement device 20. The heat treatment device 10 includes a heating block 11 and a cooling block 12. The movement device 20 includes a first actuator 21, a second actuator 22, and a container mounting portion 23.

In the following description, an XYZ orthogonal coordinate system may be set, and a positional relationship of each member may be described with reference to this XYZ orthogonal coordinate system. An X axis direction is a first horizontal direction in which the heating block 11 and the cooling block 12 are arranged. A Z axis direction is the gravity direction. A Y axis direction is a second horizontal direction orthogonal to the X axis direction and the Z axis direction (not illustrated in FIG. 2).

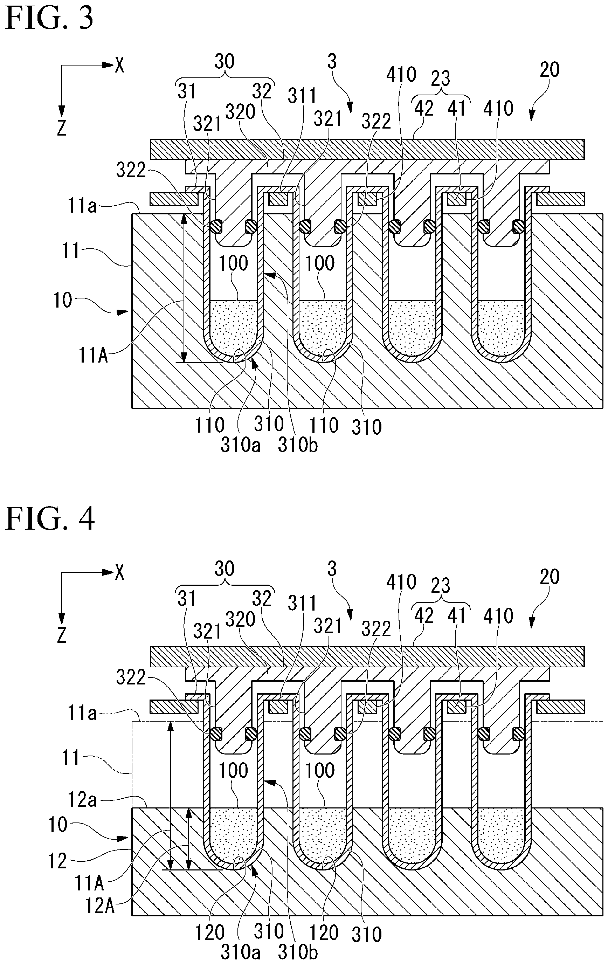

The heating block 11 heats a container 30 (refer to FIG. 3) accommodating the sample 100 described above. The cooling block 12 cools the container 30 heated by the heating block 11. The container mounting portion 23 mounts the container 30 in the movement device 20. The second actuator 22 moves the container mounting portion 23 in the Z axis direction. The first actuator 21 moves the second actuator 22 in the X axis direction.

The movement device 20 sequentially moves the container 30 mounted in the container mounting portion 23 to the heating block 11 and then the cooling block 12. The movement device 20 may be constituted to move the heat treatment device 10 (the heating block 11 and the cooling block 12) side with respect to the container 30. In addition, when the first actuator 21 performs horizontal turning instead of straight motion, the heating block 11 and the cooling block 12 may be disposed on the same radius with respect to a turning axis of the first actuator 21.

FIG. 3 is a cross-sectional view of the heating block 11 according to the first embodiment.

As illustrated in FIG. 3, first insertion holes 110 having the container 30 accommodating the sample 100 inserted therein are formed in the heating block 11. A plurality of first insertion holes 110 are formed on an upper surface 11a of the heating block 11. For example, it is favorable that the heating block 11 be made of a metal material such as copper, aluminum, or stainless steel having a high heat conductivity. For example, the heating block 11 is heated by an electrothermal heater (not illustrated), a Peltier element, or a heat medium flowing through a flow channel (not illustrated) inside the block.

The container 30 includes a container main body 31 and a lid 32. The container main body 31 includes a plurality of accommodation portions 310 accommodating the sample 100 and coupling portions 311 coupling the plurality of accommodation portions 310 to each other. Each of the accommodation portions 310 is formed to have a bottomed tubular shape and includes a hemispherical bottom portion 310a and a body portion 310b having a uniform outer diameter and extending in the Z axis direction. For example, the bottom portion 310a may be formed to have an inverted conical shape. In addition, in the bottom portion 310a, a tip having an inverted conical shape may have a curved surface such as a hemispherical shape. In addition, the body portion 310b may have an inclined shape in which the outer diameter is slightly reduced toward the lower portion in the Z axis direction, instead of a uniform outer diameter.

The plurality of accommodation portions 310 are arranged in a row in the X axis direction. The plurality of accommodation portions 310 may be arranged in a row in the Y axis direction of may be arranged in a matrix shape in both the X axis direction and the Y axis direction. The first insertion holes 110 are formed on the upper surface 11a of the heating block 11 as many as the number and in arrangement such that the plurality of accommodation portions 310 can be inserted therein in the Z axis direction. Inner wall surfaces of the first insertion holes 110 are in contact with or close to insertion parts excluding upper end portions of the accommodation portions 310. Opening edge portions of the first insertion holes 110 may be subjected to chamfering such as C-chamfering or R-chamfering such that the accommodation portions 310 are not damaged due to contact with the accommodation portions 310. The coupling portions 311 are formed to have a flat plate shape and couple the upper end portions of the plurality of accommodation portions 310 to each other.

The lid 32 includes lid portions 320 covering upper end opening portions of the plurality of accommodation portions 310, and a plurality of sealing portions 321 inserted into the upper end opening portions of the plurality of accommodation portions 310. The lid portions 320 have a flat plate shape having substantially the same size as the coupling portions 311 in a plan view. The plurality of sealing portions 321 are a plurality of projecting portions protruding downward from lower surfaces of the lid portions 320. An annular groove having a sealing material 322 disposed therein is formed on a circumferential surface of a lower end portion of each of the plurality of sealing portions 321.

The sealing material 322 is formed to have an annular shape, is inserted into the accommodation portion 310, and performs sealing by abutting an inner wall surface of each of the plurality of accommodation portions 310. The sealing material 322 is positioned in the first insertion hole 110 in a state in which each of the plurality of accommodation portions 310 is inserted into the first insertion hole 110. Accordingly, while the nucleic acid is extracted by heating, it is possible to settle a region in which a liquid and vapor of the sample 100 can be present in the first insertion hole 110. Therefore, the temperature of the sample 100 sealed in the container 30 can be minutely controlled, and therefore the nucleic acid is easily extracted.

It is desirable that the sealing materials 322 be materials having a lower modulus of elasticity than at least any of the accommodation portions 310 or the lid portions 320. Since the sealing materials 322 are deformed due to the low modulus of elasticity, the sealing portions 321 can be inserted into the upper end opening portions of the accommodation portions 310 with a smaller force. In addition, since the modulus of elasticity is low, the sealing materials 322 can reliably fill gaps between the annular grooves provided on the circumferential surfaces of the lower end portions of the sealing portions 321 and the accommodation portions 310, and therefore leakage can be prevented. Regarding a material of the sealing materials 322, nitrile rubber, styrenebutadiene rubber, silicone rubber, fluorine rubber, a perfluoroelastomer, or the like can be used.

The container mounting portion 23 includes a container support portion 41 supporting the coupling portions 311 in a state in which the plurality of accommodation portions 310 is suspended, and a container fixing portion 42 overlapping the lid portions 320, sandwiching the container 30 between the container fixing portion 42 and the container support portion 41, and provided in a manner of being able to be opened and closed with respect to the container support portion 41. The container support portion 41 is formed to have a grid shape having a plurality of penetration holes 410. The plurality of penetration holes 410 are formed in the container support portion 41 as many as the number and in arrangement such that the plurality of accommodation portions 310 can be inserted therein.

The container fixing portion 42 has a flat plate shape having substantially the same size as the container support portion 41 in a plan view. The container fixing portion 42 includes hook portions (not illustrated) capable of being engaged and disengaged with respect to the container support portion 41. The container fixing portion 42 can switch between a locked state in which the container fixing portion 42 is unable to be released with respect to the container support portion 41, and an unlocked state in which the container fixing portion 42 is able to be released with respect to the container support portion 41.

The heating block 11 heats the container 30 accommodating the sample 100 including a liquid within a first range 11A in the gravity direction. The first range 11A includes a lower portion of the container 30. The lower portion of the container 30 illustrated in FIG. 3 includes a part of the bottom portions 310a and the body portions 310b of the accommodation portions 310. For example, the lower portion of the container 30 included in at least the first range 11A may be the lowest part when the dimensions of the accommodation portions 310 in the gravity direction are divided into three parts, or may be the minimum bottom portion 310a.

In addition, the first range 11A includes a part on a side above the liquid level of the sample 100 in the container 30. In the container 30 illustrated in FIG. 3, the first range 11A is on a side above the liquid level of the sample 100 and includes a part on a side below the parts in which at least the sealing materials 322 abut the inner wall surfaces of the accommodation portions 310.

FIG. 4 is a cross-sectional view of the cooling block 12 according to the first embodiment. In FIG. 4, for comparison with the heating block 11, the heating block 11 and the upper surface 11a thereof are indicated by two-dot dashed lines.

As illustrated in FIG. 4, second insertion holes 120 having the container 30 heated by the heating block 11 inserted therein are formed in the cooling block 12. A plurality of second insertion holes 120 are formed on an upper surface 12a of the cooling block 12. For example, it is favorable that the cooling block 12 be made of a metal material such as copper, aluminum, or stainless steel having a high heat conductivity. For example, the cooling block 12 is cooled by a heat medium (refrigerant) flowing through a flow channel (not illustrated) inside the block. Alternatively, the cooling block 12 may be cooled by outside air at an ordinary temperature. In addition, the cooling block 12 may be provided with fins (not illustrated) in a part thereof such that cooling at an ordinary temperature is promoted or may be in contact with a fin member (not illustrated). Alternatively, the cooling block 12 may be cooled by a Peltier element (not illustrated).

Similar to the first insertion holes 110 described above, the second insertion holes 120 are formed on the upper surface 12a of the cooling block 12 as many as the number and in arrangement such that the plurality of accommodation portions 310 can be inserted therein in the Z axis direction. Inner wall surfaces of the second insertion holes 120 are in contact with or close to the insertion parts of the accommodation portions 310 throughout the entire region. The second insertion holes 120 are formed to be shallower than the first insertion holes 110 described above.

The cooling block 12 cools the container 30 heated by the heating block 11 within a second range 12A narrower than the first range 11A in the gravity direction. The second range 12A includes the lower portion of the container 30. Namely, the cooling block 12 cools a part of the bottom portions 310a and the body portions 310b of the accommodation portions 310. In addition, in the cooling block 12 illustrated in FIG. 4, differently from the first range 11A, the second range 12A does not include a part on a side above the liquid level of the sample 100 in the container 30.

In the cooling block 12 having the foregoing constitution, since the cooling block 12 abuts or is close to only the lower portion of the container 30, although the lower portion of the container 30 is cooled, an upper portion of the container 30 is not cooled and residual heat can be retained. For this reason, the lower portion of the container 30 and the sample 100 are cooled earlier than the upper portion of the container 30. Consequently, vapor inside the container 30 condenses in a form of being absorbed by the lower portion of the container 30 or the sample 100. For this reason, adhesion of vapor inside the container 30 to a wall surface of the upper portion of the container 30 as droplets can be curbed.

In addition, the amount of vapor inside the container 30 can be reduced compared to the amount of vapor at the time of starting cooling. For this reason, even if the upper portion of the container 30 is gradually cooled naturally, vapor can be collected in the lower portion of the container 30 without adhering to the wall surface of the upper portion of the container 30 as droplets.

As a result, adhesion of droplets to the wall surface of the container 30, particularly, the wall surface of the upper portion of the container 30 can be curbed. Therefore, it is possible to resolve problems such as reduction in the amount of sample which can be transferred to other containers, or occurrence of contamination due to scattering of droplets which have adhered to the upper portion of the container 30 at the time of opening and closing the lid 32.

In this manner, according to the first embodiment described above, the heating block 11 that heats the container 30 accommodating the sample 100 including a liquid within the first range 11A in the gravity direction, and the cooling block 12 that cools the container 30 heated by the heating block 11 within the second range 12A narrower than the first range 11A in the gravity direction are included. According to this constitution, it is possible to obtain the treatment apparatus, in which adhesion of droplets to the wall surface of the container 30 due to condensation of vapor can be curbed, the nucleic acid extraction system 3 including the treatment apparatus, and the nucleic acid analysis system 1 including the nucleic acid extraction system 3.

In addition, in the first embodiment, the second range 12A includes the lower portion of the container 30. According to this constitution, although the lower portion of the container 30 is cooled, the upper portion of the container 30 is not cooled and the residual heat can be retained. For this reason, the lower portion of the container 30 and the sample 100 are cooled earlier than the upper portion of the container 30. Consequently, vapor inside the container 30 condenses in a form of being absorbed by the lower portion of the container 30 or the sample 100. For this reason, adhesion of vapor inside the container 30 to the wall surface of the upper portion of the container 30 as droplets can be curbed.

In addition, in the first embodiment, the first insertion holes 110 into which the container 30 is inserted and heated within the first range 11A are formed in the heating block 11. The second insertion holes 120 into which the container 30 is inserted and cooled within the second range 12A are formed in the cooling block 12. The second insertion holes 120 are shallower than the first insertion holes 110. According to this constitution, the second range 12A of cooling the container 30 can be easily made narrower in the gravity direction than the first range 11A of heating the container 30.

### [Second embodiment]

Next, a second embodiment of the present invention will be described. In the following description, the same reference signs are applied to constituents which are the same as or equivalent to those of the embodiment described above, and description thereof will be simplified or omitted.

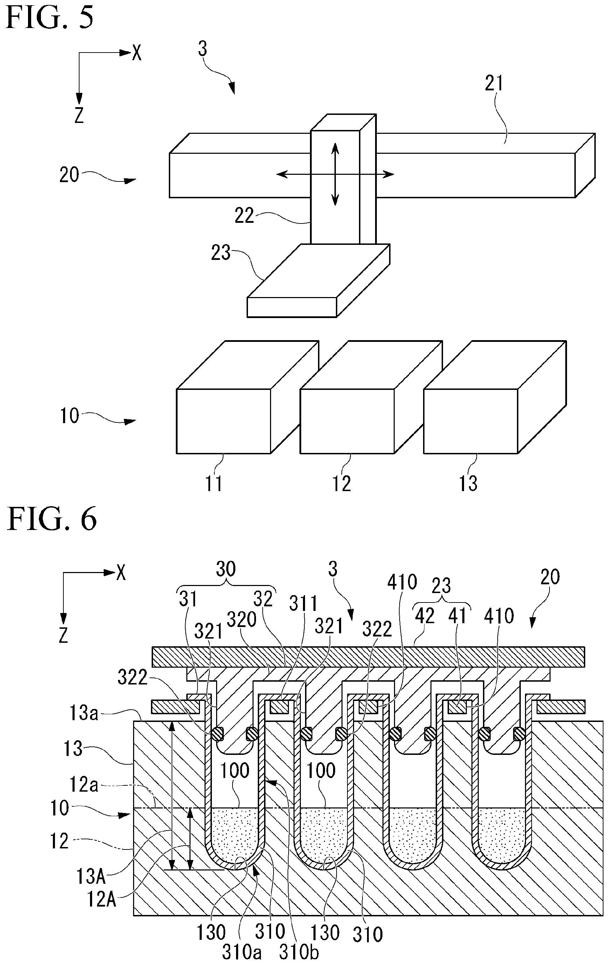

FIG. 5 is a schematic view of the nucleic acid extraction system 3 according to the second embodiment.

As illustrated in FIG. 5, the heat treatment device 10 of the nucleic acid extraction system 3 of the second embodiment includes a second cooling block 13 in addition to the heating block 11 and the cooling block 12 (also referred to as a first cooling block) described above. The movement device 20 sequentially moves the container 30 mounted in the container mounting portion 23 to the heating block 11, the cooling block 12, and then the second cooling block 13.

FIG. 6 is a cross-sectional view of the second cooling block 13 according to the second embodiment. In FIG. 6, for comparison with the cooling block 12, the cooling block 12 and the upper surface 12a thereof are indicated by two-dot dashed lines.

As illustrated in FIG. 6, third insertion holes 130 having the container 30 cooled by the cooling block 12 inserted therein are formed in the second cooling block 13. A plurality of third insertion holes 130 are formed on an upper surface 13a of the second cooling block 13. For example, it is favorable that the second cooling block 13 be made of a metal material such as copper, aluminum, or stainless steel having a high heat conductivity. For example, the second cooling block 13 is cooled by a heat medium (refrigerant) flowing through a flow channel (not illustrated) inside the block. Alternatively, the second cooling block 13 may be cooled by outside air at an ordinary temperature. In addition, the second cooling block 13 may be provided with fins (not illustrated) in a part thereof such that cooling at an ordinary temperature is promoted or may be in contact with a fin member (not illustrated). Alternatively, the second cooling block 13 may be cooled by a Peltier element (not illustrated).

Similar to the first insertion holes 110 and the second insertion holes 120 described above, the third insertion holes 130 are formed on the upper surface 12a of the second cooling block 13 as many as the number and in arrangement such that the plurality of accommodation portions 310 can be inserted therein in the Z axis direction. Inner wall surfaces of the third insertion holes 130 are in contact with or close to the insertion parts of the accommodation portions 310 throughout the entire region. The third insertion holes 130 are formed to be deeper than the second insertion holes 120 described above.

The second cooling block 13 cools the container 30 cooled by the cooling block 12 within a third range 13A wider than the second range 12A in the gravity direction. The third range 13A includes the lower portion of the container 30. In addition, in the second cooling block 13 illustrated in FIG. 6, differently from the second range 12A, the third range 13A includes a part on a side above the liquid level of the sample 100 in the container 30. Namely, similar to the first range 11A, the second cooling block 13 cools the third range 13A including the bottom portions 310a and the body portions 310b of the accommodation portions 310 substantially in their entirety.

In the foregoing constitution, after the amount of vapor inside the container 30 is reduced by cooling the lower portion of the container 30 in the cooling block 12 described above, the container 30 substantially in its entirety can be cooled in the second cooling block 13. For this reason, the temperature of the entire container 30 can be lowered faster than the case of lowering the temperature of the entire container 30 by only the cooling block 12, and therefore a user can take out the container 30.

In this manner, in the second embodiment, since the second cooling block 13 that cools the container 30 cooled by the cooling block 12 within the third range 13A wider than the second range 12A in the gravity direction is included, the temperature of the container 30 can be quickly lowered to a temperature of 50°C to 70°C at which a user can take out the container. When the container 30 and the second cooling block 13 are caused to abut or be close to each other, since the amount of vapor inside the container 30 has sufficiently decreased by abutting or being close to the cooling block 12 therebefore, droplets scarcely adhere to the wall surface of the upper portion of the container 30 due to condensation of vapor.

In addition, in the present embodiment, the third insertion holes 130 into which the container 30 is inserted and cooled within the third range 13A are formed in the second cooling block 13. The third insertion holes 130 are deeper than the second insertion holes 120. According to this constitution, the third range 13A of cooling the container 30 can be easily made wider in the gravity direction than the second range 12A of cooling the container 30.

### [Third embodiment]

Next, a third embodiment of the present invention will be described. In the following description, the same reference signs are applied to constituents which are the same as or equivalent to those of the embodiments described above, and description thereof will be simplified or omitted.

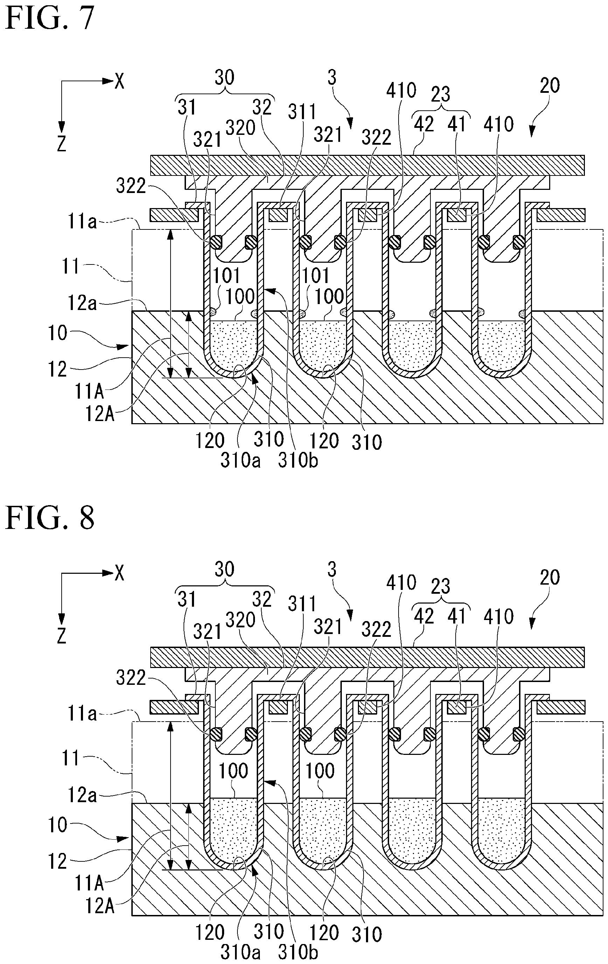

FIG. 7 is a cross-sectional view of the cooling block 12 according to the third embodiment. In FIG. 7, for comparison with the heating block 11, the heating block 11 and the upper surface 11a thereof are indicated by two-dot dashed lines.

As illustrated in FIG. 7, in the third embodiment, the second range 12A in which the cooling block 12 cools the container 30 includes a part on a side above the liquid level of the sample 100 in the container 30. The second range 12A illustrated in FIG. 7 includes the lower portion of the container 30 and a part on a side above the liquid level of the sample 100, that is, on a side below the parts in which the sealing materials 322 abut the inner wall surfaces of the accommodation portions 310.

According to this constitution, a part accommodating the sample 100 in the container 30 can be reliably cooled in the cooling block 12, and the sample 100 can be reliably and quickly cooled. In this case, droplets 101 may adhere to the wall surface of the upper portion of the container 30 due to condensation of vapor. However, if the droplets 101 are close to the liquid level of the sample 100, they can be easily collected by slightly shaking the container 30 or the like. In addition, since the droplets 101 are away from a part in the vicinity of the lid 32 in the upper portion of the container 30, there is little risk of scattering and occurrence of contamination when the lid 32 is opened.

### [Fourth embodiment]

Next, a fourth embodiment of the present invention will be described. In the following description, the same reference signs are applied to constituents which are the same as or equivalent to those of the embodiments described above, and description thereof will be simplified or omitted.

FIG. 8 is a cross-sectional view of the cooling block 12 according to the fourth embodiment. In FIG. 8, for comparison with the heating block 11, the heating block 11 and the upper surface 11a thereof are indicated by two-dot dashed lines.

As illustrated in FIG. 8, in the fourth embodiment, the second range 12Ain which the cooling block 12 cools the container 30 is on only a side below the liquid level of the sample 100 in the container 30.

According to this constitution, droplets scarcely adhere to the wall surface of the upper portion of the container 30 due to condensation of vapor. Therefore, compared to the third embodiment described above, although the cooling speed of the sample 100 is slightly slowed down, it is possible to prevent droplets from adhering to the wall surface of the container 30. Accordingly, a motion of agitating the container 30 after cooling is no longer necessary, and therefore a risk of contamination can be further reduced.

Hereinabove, preferable embodiments of the present invention have been described with reference to the drawings, but the present invention is not limited to the foregoing embodiments. Various shapes, combinations, and the like of the constituent members described in the foregoing embodiments are examples and can be variously changed based on design requirements and the like within a range not departing from the gist of the present invention.

A part or the entirety of the treatment apparatus according to the embodiments described above can be appended as follows.

### (Appendix)

A treatment apparatus includes a heating block in which first insertion holes are formed, a cooling block in which second insertion holes shallower than the first insertion holes are formed, and a movement device which moves a treatment target from the first insertion holes to the second insertion holes.

In this specification, the terms such as "front, rear, up, down, right, left, perpendicular, horizontal, vertical, lateral, rows, and columns" indicating directions are mentioned regarding the directions thereof in the apparatus of the present invention. Therefore, these terms in the specification of the present invention should be relatively interpreted in regard to the apparatus of the present invention.

The term "constituted" is used in a case of being constituted to execute the functions of the present invention or used for indicating a constitution, an element, and a part of the apparatus.

Moreover, the terms expressed as "means-plus-function" in the claims should include diverse structures which can be utilized to execute the functions included in the present invention.

The term "unit" is used for indicating a part of a constituent element, a unit, hardware, or software programmed to execute a desired function. A representative example of hardware is a device or a circuit, but it is not limited to these.

Hereinabove, preferable examples of the present invention have been described, but the present invention is not limited to these examples. Addition, omission, replacement, and other changes of the constituents can be made within a range not departing from the gist of the present invention. The present invention is not limited by the foregoing description and is limited by only the accompanying claims.

### [Reference Signs List]

1 Nucleic acid analysis system
3 Nucleic acid extraction system
11 Heating block
11A First range
12 Cooling block
12A Second range
13 Second cooling block
13A Third range
30 Container
100 Sample
110 First insertion hole
120 Second insertion hole
130 Third insertion hole

## Claims

1. A treatment apparatus comprising:
a heating block configured to heat a container accommodating a sample including a liquid within a first range in a gravity direction; and
a cooling block configured to cool the container heated by the heating block within a second range narrower than the first range in the gravity direction.

2. The treatment apparatus according to claim 1, wherein the second range includes a lower portion of the container.

3. The treatment apparatus according to claim 1 or 2, wherein the second range includes a part on a side above a liquid level of the sample in the container.

4. The treatment apparatus according to claim 1 or 2, wherein the second range is on only a side below a liquid level of the sample in the container.

5. The treatment apparatus according to any one of claims 1 to 4,
wherein a first insertion hole into which the container is inserted and heated within the first range is formed in the heating block,
wherein a second insertion hole into which the container is inserted and cooled within the second range is formed in the cooling block, and
wherein the second insertion hole is shallower than the first insertion hole.

6. The treatment apparatus according to any one of claims 1 to 5, further comprising a second cooling block configured to cool the container cooled by the cooling block within a third range wider than the second range in the gravity direction.

7. The treatment apparatus according to claim 5, further comprising a second cooling block configured to cool the container cooled by the cooling block within a third range wider than the second range in the gravity direction,
wherein a third insertion hole into which the container is inserted and cooled within the third range is formed in the second cooling block, and
wherein the third insertion hole is deeper than the second insertion hole.

8. The treatment apparatus according to claim 5, wherein the container comprises:
a container main body which has a plurality of accommodation portions accommodating the sample; and
coupling portions coupling the plurality of accommodation portions to each other.

9. The treatment apparatus according to claim 8, wherein an inner wall surface of the first insertion hole is in contact with or close to an insertion part excluding upper end portions of the plurality of accommodation portions.

10. The treatment apparatus according to claim 8 or 9, wherein the container comprises a lid which has a lid portion covering upper end opening portions of the plurality of accommodation portions and a plurality of sealing portions inserted into the upper end opening portions.

11. The treatment apparatus according to claim 10, wherein the lid portion has a flat plate shape having the same size as the coupling portions.

12. The treatment apparatus according to claim 10 or 11, wherein the plurality of sealing portions are a plurality of projecting portions protruding downward from a lower surface of the lid portion.

13. The treatment apparatus according to claim 12, wherein an annular groove having a sealing material disposed therein is formed on a circumferential surface of a lower end portion of each of the plurality of sealing portions.

14. The treatment apparatus according to claim 13, wherein the sealing material seals by abutting an inner wall surface of each of the plurality of accommodation portions.

15. The treatment apparatus according to claim 14, wherein the sealing material is a material having a lower modulus of elasticity than at least one of the plurality of accommodation portions or the lid portion.

16. The treatment apparatus according to claim 10, further comprising a movement device configured to sequentially move the container to the heating block and then the cooling block,
wherein the movement device comprises a container mounting portion in which the container is mounted, and
wherein the container mounting portion comprises:
a container support portion configured to support the coupling portions in a state in which the plurality of accommodation portions are suspended; and
a container fixing portion configured to overlap the lid portion, the container fixing portion being configured to sandwich the container together with the container support portion, and the container fixing portion being provided in a manner of being able to be opened and closed with respect to the container support portion.

17. The treatment apparatus according to claim 16, wherein the container fixing portion has a flat plate shape having the same size as the container support portion.

18. The treatment apparatus according to claim 16 or 17, wherein the container fixing portion comprises a hook portion capable of being engaged and disengaged with respect to the container support portion, and
wherein the container fixing portion switches between a locked state in which the container fixing portion is unable to be released with respect to the container support portion, and an unlocked state in which the container fixing portion is able to be released with respect to the container support portion.

19. A nucleic acid extraction system comprising the treatment apparatus according to any one of claims 1 to 18, the nucleic acid extraction system extracting nucleic acid from cells of the sample.

20. A nucleic acid analysis system comprising the nucleic acid extraction system according to claim 19, the nucleic acid analysis system analyzing the extracted nucleic acid.
